(19) Europäisches Patentamt
European Patent Office
Office européen des brevets

(11) Veröffentlichungsnummer: **0 087 097**
**B1**

(12) # EUROPÄISCHE PATENTSCHRIFT

(45) Veröffentlichungstag der Patentschrift: **13.05.87**

(51) Int. Cl.⁴: **C 07 C 69/18**, C 07 C 69/145, C 07 C 67/05

(21) Anmeldenummer: **83101388.3**

(22) Anmeldetag: **14.02.83**

(54) **2-Alkyl-1,3-diacyloxy-4-hydroxy-1-butene, ihre Herstellung und Verwendung.**

(30) Priorität: **20.02.82 DE 3206135**

(43) Veröffentlichungstag der Anmeldung: **31.08.83 Patentblatt 83/35**

(45) Bekanntmachung des Hinweises auf die Patenterteilung: **13.05.87 Patentblatt 87/20**

(84) Benannte Vertragsstaaten: **CH DE FR GB IT LI**

(56) Entgegenhaltungen:
**EP - A - 0 005 452**
**EP - A - 0 010 656**
**EP - A - 0 068 372**
**DE - A - 2 842 238**

(73) Patentinhaber: **BASF Aktiengesellschaft, Carl-Bosch-Strasse 38, D-6700 Ludwigshafen (DE)**

(72) Erfinder: **Fischer, Rolf-Hartmuth, Dr., Bergstrasse 98, D-6900 Heidelberg (DE)**
Erfinder: **Weitz, Hans Martin, Dr., Auf dem Koeppel 40, D-6702 Bad Duerkheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue 2-Alkyl-1,3-diacyloxy-4-hydroxy-1-butene, ein Verfahren zu ihrer Herstellung durch Umsetzung von 1-Acyloxy-2-alkyl-1,3-butadienen mit Carbonsäuren und Sauerstoff sowie die Verwendung der neuen 2-Alkyl-1,3-diacyloxy-4-hydroxy-1-butene für die Herstellung von 2-Alkyl-4-acyloxy-2-butenalen.

Es ist aus der EP-PS 5 452 bekannt, dass bei der Umsetzung von 1-Acyloxy-2-alkyl-1,3-butadienen mit Carbonsäuren und Sauerstoff in Gegenwart von Palladium oder Platin enthaltenden Katalysatoren 2-Alkyl-1,1,4-triacyloxy-2-butene entstehen, die sich zu 2-Alkyl-4-acyloxy-2-butenalen verseifen lassen.

Es wurde nun gefunden, dass man neue 2-Alkyl-1,3-diacyloxy-4-hydroxy-1-butene der Formel

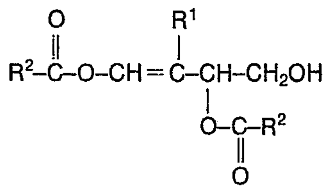

I,

in der $R^1$ einen Alkylrest mit 1 bis 5 Kohlenstoffatomen und $R^2$ ein Wasserstoffatom, einen Alkylrest mit 1 bis 5 Kohlenstoffatomen, einen cycloaliphatischen Rest mit 5 bis 7 Kohlenstoffatomen oder einen aromatischen Rest bedeuten, erhält, wenn man 1-Acyloxy-2-alkyl-1,3-butadiene der Formel

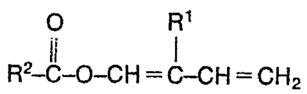

III,

in der $^1$ und $R^2$ die oben genannte Bedeutung haben, in Gegenwart von Sauerstoff oder von Sauerstoff abgebenden Verbindungen und in Abwesenheit von Palladium oder Platin enthaltenden Katalysatoren mit Carbonsäuren der Formel $R^2$–COOH, in der $R^2$ die oben genannte Bedeutung hat, umsetzt.

Bei dem erfindungsgemässen Verfahren entstehen die neuen
2-Alkyl-1,3-diacyloxy-4-hydroxy-1-butene
der Formel I neben
2-Alkyl-1,1-diacyloxy-4-hydroxy-2-butenen
der Formel

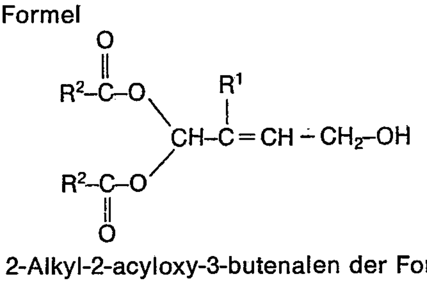

IV,

und 2-Alkyl-2-acyloxy-3-butenalen der Formel

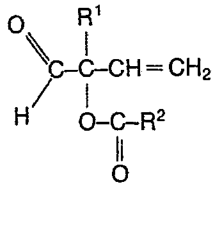

V.

In den Formeln IV und V haben die Reste $R^1$ und $R^2$ die obengenannte Bedeutung.

Von den Verbindungen der Formel I sind die 2-Alkyl-1,3-diacyloxy-4-hydroxy-1-butene der Formel

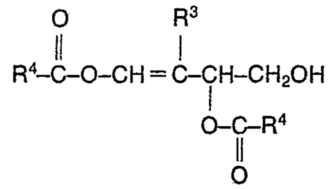

II,

in der $R^3$ einen Alkylrest mit 1 bis 3 Kohlenstoffatomen und $R^4$ ein Wasserstoffatom oder einen Alkylrest mit 1 bis 3 Kohlenstoffatomen bedeutet, von besonderem technischen Interesse. Besonders bevorzugt ist das 2-Methyl-1,3-diacetoxy-4-hydroxy-1-buten.

In den Ausgangsstoffen der Formel III steht $R^1$ für einen Alkylrest mit 1 bis 5, vorzugsweise 1 bis 3 Kohlenstoffatomen. $R^2$ bedeutet ein Wasserstoffatom, einen Alkylrest mit 1 bis 5, vorzugsweise 1 bis 3 Kohlenstoffatomen, einen cycloaliphatischen Rest, z. B. einen Cyclopentyl-, Cyclohexyl- oder Cycloheptylrest oder einen aromatischen Rest, z. B. einen Phenylrest, der durch Alkylreste oder Halogenatome substituiert sein kann. Alkylreste sind z. B. Methyl-, Ethyl-, n-Propyl-, i-Propyl-, n-Butyl-, i-Butyl-, tert.-Butyl- oder n-Pentylreste.

Die als Ausgangsstoffe zu verwendenden 1-Acyloxy-2-alkyl-1,3-butadiene sind durch Acylierung von $\alpha,\beta$-ungesättigten Aldehyden mit Säureanhydriden, Säurehalogeniden, Ketenen oder Enolestern zugänglich (Houben-Weyl, Methoden der organischen Chemie, 4. Auflage, Band V, 1c, Seiten 184 bis 192). So ist 1-Acetoxy-2-methyl-1,3-butadien z. B. aus Tiglinaldehyd (2-Methylcrotonaldehyd) oder 2-Ethylacrolein herstellbar (Ind. Eng. Chem. 41, 2920 (1949)).

Beispielsweise seien die folgenden Verbindungen der Formel III genannt:

2-Methyl-, 2-Ethyl-, 2-n-Propyl-, 2-Butyl-,
2-Pentyl-1-acetoxy-1,3-butadien,
2-Methyl-1-propionyloxy-1,3-butadien,
2-Methyl-1-cyclohexyloxy-1,3-butadien und
2-Methyl-1-benzoyloxy-1,3-butadien.

Als Carbonsäuren der Formel $R^2$–COOH kommen z. B. Ameisensäure, Essigsäure, Propionsäure, Buttersäure, Valeriansäure, Caprinsäure, Laurinsäure, Ölsäure, Palmitinsäure, Cyclohexancarbonsäure, Benzoesäure oder Phenylessigsäure in Betracht. Essigsäure ist aus wirtschaftlichen Gründen bevorzugt. Die Carbonsäure wird im allgemeinen im Überschuss, z. B. in der 1 bis 80molaren Menge, bezogen auf das eingesetzte 1,3-Dien III angewandt.

Sauerstoff kann in Form von reinem Sauerstoff oder im Gemisch mit anderen Gasen, wie Stickstoff z. B. in Form von Luft oder mit anderen inerten Gasen, wie Kohlendioxid verwendet werden. Als Sauerstoff abgebende Verbindungen sind z. B. solche geeignet, die für die Epoxidierung von Olefinen verwendet werden, wie Wasserstoffperoxid,

Persäuren, Perameisensäure, Peressigsäure, Perpropionsäure, Perbenzoesäure, Per-n-buttersäure, Per-iso-buttersäure und organische Hydroperoxide, wie tert.-Butylhydroperoxid oder Cumolhydroperoxid. Verbindungen dieser Art sind z.B. in Ullmanns Encyclopädie der Technischen Chemie, 4. Auflage, Band 10, Seiten 563 bis 567 genannt.

Das erfindungsgemässe Verfahren sei am Beispiel der Umsetzung von
1-Acetoxy-2-methyl-1,3-butadien
in Essigsäure mit Peressigsäure zu
2-Methyl-1,3-diacetoxy-4-hydroxy-1-buten,
2-Methyl-1,1-diacetoxy-4-hydroxy-2-buten und
2-Methyl-2-acetoxy-3-butenal
durch die folgenden Formeln erläutert:

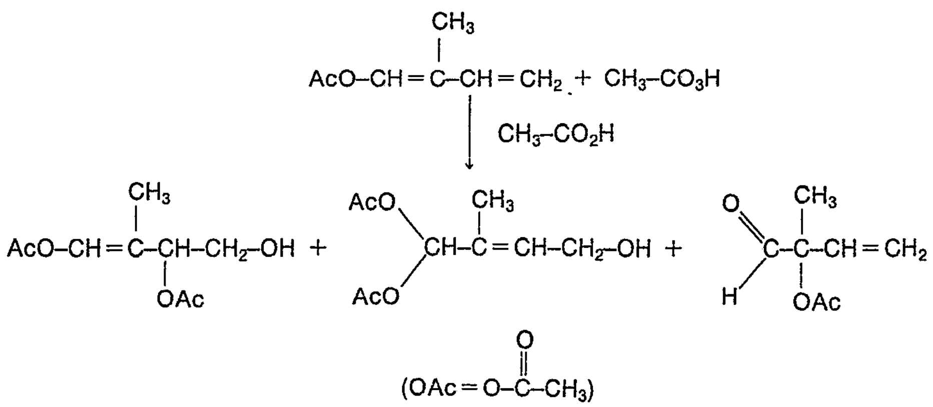


Im einzelnen wird das Verfahren z.B. so durchgeführt, dass man pro Mol 1,3-Dien der Formel III 1 bis 80 Mol, insbesondere 1,5 bis 60 Mol der Carbonsäure und 0,5 bis 5 Mol, insbesondere 1 bis 1,5 Mol an Sauerstoff oder der Sauerstoff abgebenden Verbindung anwendet. Man arbeitet zweckmässig bei Temperaturen zwischen 0 und 200 °C, insbesondere bei 20 bis 120 °C und wendet z.B. Sauerstoffdrucke von 1 bis 100, insbesondere 1 bis 20 bar an.

Das Verfahren kann chargenweise oder kontinuierlich, drucklos oder unter Druck durchgeführt werden. Unumgesetzte 1,3-Diene (III) können gegebenenfalls nach der Umsetzung destillativ von den entstandenen Wertprodukten abgetrennt und erneut für die erfindungsgemässe Umsetzung verwendet werden.

Gewöhnlich arbeitet man entweder in einem Überschuss der Carbonsäure als Lösungsmittel oder mit geringeren Mengen an Carbonsäure und in Gegenwart von unter den Reaktionsbedingungen inerten Lösungsmitteln. Als Lösungsmittel dieser Art kommen z.B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole, Ether, wie Diethylether, Tetrahydrofuran oder Dioxan in Betracht. Pro Mol Ausgangsverbindung (III) verwendet man zweckmässigerweise 0,1 bis 80 Mol, insbesondere 2 bis 60 Mol, des unter den Reaktionsbedingungen inerten Lösungsmittels.

Die Umsetzung kann man bei diskontinuierlicher Arbeitsweise, z.B. wie folgt vornehmen: Einer Lösung des 1,3-Diens (III) in der Carbonsäure, die gegebenenfalls noch das Lösungsmittel enthält, werden bei der Reaktionstemperatur und dem Reaktionsdruck Sauerstoff oder die Sauerstoff abgebende Verbindung zugeführt. Nach beendeter Zugabe wird gegebenenfalls noch nachgerührt. Durch das auf Raumtemperatur abgekühlte Reaktionsgemisch wird gegebenenfalls Stickstoff geleitet. Anschliessend wird nach dem Abdestillieren des Lösungsmittels und/oder der Carbonsäure fraktioniert destilliert. Dabei werden gegebenenfalls nicht umgesetzte Ausgangsverbindungen von den gewünschten Produkten abgetrennt.

Die erfindungsgemässen
2-Alkyl-1,3-diacyloxy-4-hydroxy-1-butene (I)
und die nach dem erfindungsgemässen Verfahren erhältlichen
2-Alkyl-1,1-diacyloxy-4-hydroxy-2-butene (IV)
und 2-Alkyl-2-acyloxy-3-butenale (V)
sind wertvolle Zwischenprodukte, z.B. für die Herstellung von biologisch aktiven Verbindungen.

Die neuen
2-Alkyl-1,3-diacyloxy-4-hydroxy-1-butene
werden erfindungsgemäss für die Herstellung von 2-Alkyl-4-acyloxy-2-butenalen verwendet. Von diesen Verbindungen dient z.B. das 2-Methyl-4-acetoxy-2-butenal als Vorprodukt für die Herstellung von Vitamin A und seiner Abkömmlinge. So lässt sich beispielsweise aus 2-Methyl-1,3-diacetoxy-4-hydroxy-1-buten das gesuchte 2-Methyl-4-acetoxy-2-butenal durch Behandlung mit Acetylierungsmitteln, wie Acetanhydrid, in saurem Milieu herstellen (s. Beispiel 3). Führt man die Behandlung mit den Acetylierungsmitteln in basischem Milieu (s. Beispiel 4) durch, so werden Triacetoxy-2-methyl-2-butene gebildet, die z.B. mit Wasser in Gegenwart von Carbonsäuren, wie das 1,1,4-Triacetoxy-2-methyl-2-buten (ds. EP-PS 10 656) zu 2-Methyl-4-acetoxy-2-butenal umgesetzt werden. Da das 2-Methyl-1,1-diacetoxy-4-hydroxy-2-buten bei der sauren oder basischen Acylierung mit gleichem Ergebnis wie das 2-Methyl-1,3-diacetoxy-4-hydroxy-1-buten reagiert, ist es nicht erforderlich, dieses Nebenprodukt vorher abzutrennen. Die Erfindung eröffnet somit einen neuen Weg für die Herstellung dieses wichtigen Vorproduktes für Vitamin-A.

Beispiel 1

Umsetzung von 1-Acetoxy-2-methyl-1,3-butadien mit Essigsäure und Peressigsäure

Zu einer Lösung von 126 g 1-Acetoxy-2-methyl-1,3-butadien in 300 g Eisessig wurden bei 30 ± 2 °C unter Rühren und Kühlung innerhalb von 2 Stunden 782 g Peressigsäure in Eisessig (12,2 Gew.%, hergestellt nach Houben-Weyl, 4. Auflage, Methoden der organischen Chemie, Band 8, Seite 41) gegeben. Man rührte 18 Stunden bei Raumtemperatur. Peroxid konnte nach dieser Zeit nicht mehr nachgewiesen werden. Aus dem Reaktionsaustrag (1210 g) wurde die Hauptmenge der Essigsäure am Rotationsverdampfer bei einer Badtemperatur von 30 bis 40 °C und einem Druck von 20 mbar abgezogen. Es wurden 297 g einer gelblichen Flüssigkeit erhalten. Diese wurde in einem Liter Ether aufgenommen. Die Etherphase wurde dreimal mit je 100 $cm^3$ einer gesättigten wässrigen Natriumbicarbonatlösung ausgeschüttelt. Die Etherphase wurde über Magnesiumsulfat getrocknet und der Ether am Rotationsverdampfer abgezogen.

Aus dem Rückstand (129 g) wurden durch fraktionierte Destillation 38 g 2-Methyl-2-acetoxy-3-butenal vom Siedepunkt 36 bis 40 °C/0,3 mbar, $n_D^{20}$ = 1,4330 (27%, bezogen auf eingesetztes 1-Acetoxy-2-methyl-1,3-butadien) erhalten.

$^1$H-NMR-Spektrum (Lösungsmittel $CDCl^3$, TMS als innerer Standard): δ = 1,55 (s, 3H), 2,14 (s, 3H), 5,1–5,6 (m, 2H), 5,6–6,2 (m, 2H), 9,30 ppm (s, 1H).

Der bei der fraktionierten Destillation erhaltene Rückstand (88,5 g) wurde im Kugelrohr bei 0,1 mbar und einer Badtemperatur von 170 °C destilliert. Man erhielt 75,7 g eines Gemisches vom Brechungsindex $n_D^{20}$ = 1,4592, das aufgrund seines NMR-Spektrums zu etwa gleichen Teilen aus 2-Methyl-1,3-diacetoxy-4-hydroxy-1-buten und 2-Methyl-1,1-diacetoxy-4-hydroxy-2-buten bestand (37%, bezogen auf eingesetztes 1-Acetoxy-2-methyl-1,3-butadien).

$^1$ H-NMR-Spektren (Lösungsmittel $CDCl^3$, TMS als innerer Standard): 2-Methyl-1,3-diacetoxy-4-hydroxy-1-buten: δ = 1,70 (s, 3H), 2,1 (s, 6H), 3,65 (d, 2H), 5,20 (t, 1H), 7,14 ppm (s, 1H). 2-Methyl-1,1-diacetoxy-4-hydroxy-2-buten: δ = 1,70 (s, 3H), 2,1 (s, 6H), 4,16 (d, 2H), 5,83 (t, 1H), 6,95 ppm (s, 1H).

Beispiel 2

Umsetzung von 1-Acetoxy-2-methyl-1,3-butadien mit Essigsäure und Luftsauerstoff

Eine Lösung von 10 g 1-Acetoxy-2-methyl-1,3-butadien in 200 g Eisessig wurde 40 Stunden bei 100 °C unter Luftzutritt mit einem Begasungsrührer gerührt. Nach Abziehen der Essigsäure erhielt man durch fraktionierte Destillation 1,9 g 2-Methyl-2-acetoxy-3-butenal vom Siedepunkt 39 °C/0,5 mbar, $n_D^{20}$ = 1,4339 (17%, bezogen auf eingesetztes 1-Acetoxy-2-methyl-1,3-butadien).

Beispiel 3

Verwendung von 2-Methyl-1,3-diacetoxy-4-hydroxy-1-buten für die Herstellung von 2-Methyl-4-acetoxy-2-butenal

8,7 g eines nach Beispiel 1 hergestellten Gemisches aus
2-Methyl-1,3-diacetoxy-4-hydroxy-1-buten und
2-Methyl-1,1-diacetoxy-4-hydroxy-2-buten,
gelöst in 100 g Eisessig wurden mit 5 g Acetanhydrid und 0,1 g p-Toluolsulfonsäure versetzt und 2 Stunden auf 100 °C erhitzt. Nach Abziehen der Essigsäure am Rotationsverdampfer und fraktionierter Destillation des Rückstandes erhielt man 5,5 g 2-Methyl-4-acetoxy-2-butenal vom Siedepunkt 62 °C/0,5 mbar, $n_D^{20}$ = 1,4622 (90%, bezogen auf eingesetztes 2-Methyl-diacetoxy-4-hydroxybuten).

Beispiel 4

Verwendung von 2-Methyl-1,3-diacetoxy-4-hydroxy-buten für die Herstellung von 2-Methyl-4-acetoxy-2-butenal

8,7 g eines nach Beispiel 1 hergestellten Gemisches aus
2-Methyl-1,3-diacetoxy-4-hydroxy-1-buten und
2-Methyl-1,1-diacetoxy-4-hydroxy-2-buten
wurden in 100 $cm^3$ Pyridin gelöst. Es wurden 4,7 g Acetylchlorid zugegeben und bei 35 °C 3 Stunden gerührt. Nach Abziehen von Essigsäure und Pyridin am Rotationsverdampfer erhielt man durch Kugelrohrdestillation (130 °C, 0,05 mbar) 9,2 g eines Gemisches, das nach dem NMR-Spektrum zu etwa gleichen Teilen aus
2-Methyl-1,3,4-triacetoxy-1-buten und 2-Methyl-1,1,4-triacetoxy-2-buten (87%, bezogen auf eingesetztes 2-Methyl-diacetoxy-4-hydroxy-buten) bestand.

$^1$H-NMR-Spektrum (Lösungsmittel $CDCl_3$, TMS als innerer Standard): 2-Methyl-1,3,4-triacetoxy-1-buten: δ = 1,75 (s, 3H), 2,1 (s, 9H), 4,18 (d, 2H), 5,40 (t, 1H), 7,20 ppm (s, 1H).

5,4 g dieses 2-Methyl-triacetoxybuten-Gemisches wurden mit 60 g Essigsäure und 2,2 g Wasser versetzt und 6,5 Stunden auf 100 °C erhitzt. Nach Abziehen von Essigsäure und Wasser am Rotationsverdampfer erhielt man durch Kugelrohrdestillation (110 °C, 0,1 mbar) 2,5 g 2-Methyl-4-acetoxy-2-butenal vom Brechungsindex $n_D^{20}$ = 1,4640 (79%, bezogen auf eingesetzte 2-Methyltriacetoxybutene).

**Patentansprüche**

1. 2-Alkyl-1,3-diacyloxy-4-hydroxy-1-butene der Formel

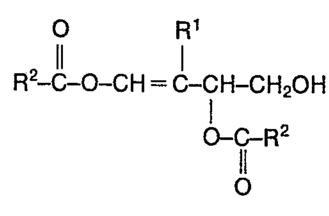
I,

in der R¹ einen Alkylrest mit 1 bis 5 Kohlenstoffatomen und R² eine Wasserstoffatom, einen Alkylrest mit 1 bis 5 Kohlenstoffatomen, einen cycloaliphatischen Rest mit 5 bis 7 Kohlenstoffatomen oder einen aromatischen Rest bedeuten.

2. 2-Alkyl-1,3-diacyloxy-4-hydroxy-1-butene der Formel

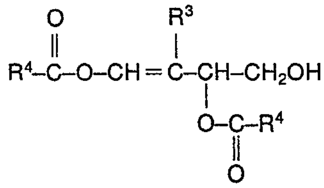

II,

in der $R^3$ einen Alkylrest mit 1 bis 3 Kohlenstoffatomen und $R^4$ ein Wasserstoffatom oder einen Alkylrest mit 1 bis 3 Kohlenstoffatomen bedeutet.

3. 2-Methyl-1,3-diacetoxy-4-hydroxy-1-buten.

4. Verfahren zur Herstellung der 2-Alkyl-1,3-diacyloxy-4-hydroxy-1-butene gemäss Anspruch 1, dadurch gekennzeichnet, dass man 1-Acyloxy-2-alkyl-1,3-butadiene der Formel

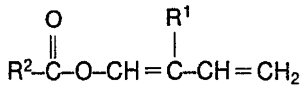

III,

in der $R^1$ und $R^2$ die in Anspruch 1 genannte Bedeutung haben, in Gegenwart von Sauerstoff oder von Sauerstoff abgebenden Verbindungen und in Abwesenheit von Palladium oder Platin enthaltenden Katalysatoren mit Carbonsäuren der Formel $R^2$–COOH, in der $R^2$ die obengenannte Bedeutung hat, umsetzt.

5. Verwendung der 2-Alkyl-1,3-diacyloxy-4-hydroxy-1-butene gemäss Anspruch 1 für die Herstellung von 2-Alkyl-4-acyloxy-2-butenalen.

**Claims**

1. A 2-alkyl-1,3-diacyloxy-4-hydroxybut-1-ene of the formula

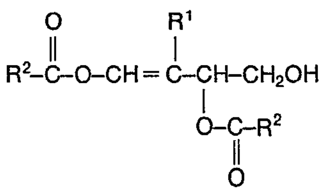

I,

where $R^1$ is alkyl of 1 to 5 carbon atoms, and $R^2$ is hydrogen, alkyl of 1 to 5 carbon atoms, a cycloaliphatic radical of 5 to 7 carbon atoms or an aromatic radical.

2. A 2-alkyl-1,3-diacyloxy-4-hydroxybut-1-ene of the formula

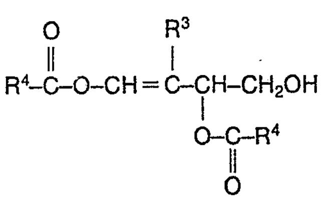

II,

where $R^3$ is alkyl of 1 to 3 carbon atoms, and $R^4$ is hydrogen or alkyl of 1 to 3 carbon atoms.

3. 2-Methyl-1,3-diacetoxy-4-hydroxybut-1-ene.

4. A process for the preparation of a 2-alkyl-1,3-diacyloxy-4-hydroxybut-1-ene as claimed in claim 1, wherein a 1-acyloxy-2-alkylbutadiene-1,3 of the formula

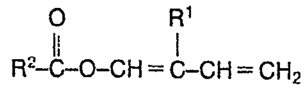

III,

where $R^1$ and $R^2$ have the meanings given in claim 1, is reacted with a carboxylic acid of the formula $R^2$–COOH, where $R^2$ has the above meanings, in the presence of oxygen or an oxygendonating compound and in the absence of a catalyst containing palladium or platinum.

5. The use of 2-alkyl-1,3-diacyloxy-4-hydroxybut-1-enes as claimed in claim 1 for the production of 2-alkyl-4-acyloxybut-2-enals.

**Revendications**

1. 2-alkyl-1,3-diacyloxy-4-hydroxy-1-butènes de formule

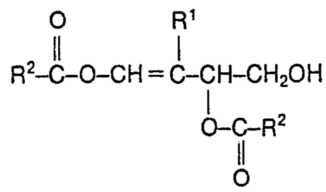

I,

dans laquelle $R^1$ représente un radical alkyle à 1–5 atomes de carbone et $R^2$ représente un atome d'hydrogène, un radical alkyle à 1–5 atomes de carbone, un radical cycloaliphatique à 5–7 atomes de carbone ou un radical aromatique.

2. 2-alkyl-1,3-diacyloxy-4-hydroxy-1-butènes de formule

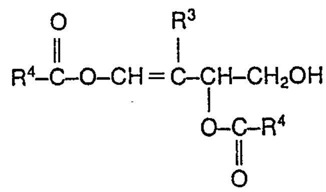

II,

dans laquelle $R^3$ représente un radical alkyle à 1–3 atomes de carbone et $R^4$ représente un atome d'hydrogène ou un radical alkyle à 1–3 atomes de carbone.

3. 2-méthyl-1,3-diacétoxy-4-hydroxy-1-butène.

4. Procédé de préparation des 2-alkyl-1,3-diacyloxy-4-hydroxy-1-butènes selon la revendication 1, caractérisé en ce qu'on fait réagir des 1-acyloxy-2-alkyl-1,3-butadiènes de formule

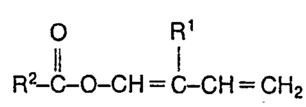

III,

dans laquelle $R^1$ et $R^2$ ont les significations données dans la revendication 1, en présence d'oxygène ou de composés délivrant de l'oxygène et en l'absence de catalyseurs contenant du palladium ou du platine, avec des acides carboxyliques de formule $R^2$–COOH, dans laquelle $R^2$ a la signification donnée ci-dessus.

5. Utilisation des 2-alkyl-1,3-diacyloxy-4-hydroxy-1-butènes selon la revendication 1 pour la préparation de 2-alkyl-4-acyloxy-2-buténals.